# EUROPEAN PATENT SPECIFICATION

(11) **EP 3 202 793 B1**
(45) Date of publication and mention of the grant of the patent: **08.05.2019**
(21) Application number: 15846767.0
(22) Date of filing: 30.09.2015
(51) Int. Cl.: C08F 2/01, C08F 2/38, C08F 20/00, C08L 33/02, C08L 33/04, C08L 33/12

(54) **METHOD FOR PRODUCING (METH)ACRYLIC RESIN**
VERFAHREN ZUR HERSTELLUNG VPM (METH)ACRYLHARZ
PROCÉDÉ DE PRODUCTION D'UNE RÉSINE (MÉTH)ACRYLIQUE

(30) Priority: 30.09.2014 JP 2014199713
(43) Date of publication of application: 09.08.2017
(73) Proprietor: Kuraray Co., Ltd., Kurashiki-shi, Okayama 710-0801 (JP)
(72) Inventor: OZAWA, Hiroshi, Tainai-shi Niigata 959-2691 (JP); KITADE, Yasuhito, Tainai-shi Niigata 959-2691 (JP); TANAKA, Shouji, Tainai-shi Niigata 959-2691 (JP)
(74) Representative: Müller-Boré & Partner Patentanwälte PartG mbB
(86) International application number: PCT/JP2015/077695
(87) International publication number: WO 2016/052600

(56) References cited:
- JP-A- 2001 187 761
- JP-A- 2005 314 471
- JP-A- 2007 197 519
- JP-A- 2009 215 359
- JP-A- 2012 087 241
- JP-A- 2013 507 467

## Description

### TECHNICAL FIELD

The present invention relates to a method for producing a (meth) acrylic resin. The present invention more specifically relates to a method for producing a (meth) acrylic resin at a low cost while maintaining high transparency even in long-term production using a polymerization apparatus.

### BACKGROUND ART

Shaped products made of a (meth) acrylic resin are excellent in transparency and cause less optical distortion, they are broadly used as optical members such as lenses, prisms, phase difference films, light guide plates, light diffusion films, polarizing plate protective films and the like.

For industrial production of (meth)acrylic resins, used is aa polymerization apparatus equipped with a raw material tank, a pipe, a pump, a stirrer, a reactor and so on. Chemical equipment such as a polymerization apparatus and the like are generally produced with anticorrosion steel materials. For example, patent document 1 discloses a mercaptan reaction vessel made if an aluminum-coated stainless steel. Patent document 2 discloses a mercaptan reaction vessel made of a stainless steel comprising Nb or Ti. However, when long-term production is performed using such a polymerization apparatus, the obtained (meth)acrylic resin can be gradually colored and the transparency can decrease.

### CITATION LIST

### PATENT LITERATURES

Patent Document 1 : JP2004-10592 A
Patent Document 2 : JP2004-59974 A

### SUMMARY OF THE INVENTION

### PROBLEMS TO BE SOLVED BY THE INVENTION

An object of the present invention is to provide a method for producing a (meth)acrylic resin at a low cost while maintaining high transparency even in long-term production using a polymerization apparatus.

### MEANS FOR SOLVING THE PROBLEMS

As a result of intensive research to achieve the above object, when a tank for storing a thiol chain transfer agent or a pipe for transferring a thiol chain transfer agent is manufactured using an austenitic stainless steel with a Mo content of 0.5 to 7.0 % by mass, it has been found out that a (meth) acrylic resin can be produced while maintaining high transparency even in long-term production. The present invention has been completed based on the finding.

Specifically, the present invention encompasses the following aspects.
[1] A method for producing (meth)acrylic resin, the method comprising storing a thiol chain transfer agent in a tank made of an austenitic stainless steel with a Mo content of 0.5 to 7.0 % by mass, transferring the thiol chain transfer agent to a polymerization reactor via a pipe made of an austenitic stainless steel with a Mo content of 0.5 to 7.0 % by mass, and radical polymerizing methyl methacrylate in the polymerization reactor.
[2] The method for producing (meth)acrylic resin according to the aspect [1], wherein, in the radical polymerizing, an acrylic acid alkyl ester is radical-polymerized together with the methyl methacrylate.
[3] The method for producing (meth)acrylic resin according to the aspect [1] or [2], wherein the tank and the pipe are made of an austenitic stainless steel with a Cr content of 16 to 18 % by mass, a Ni content of 10 to 14 % by mass, a Mo content of 2 to 3 % by mass, a C content of not more than 0.08 % by mass, a Si content of not more than 1 % by mass, a Mn content of not more than 2 % by mass, a P content of not more than 0.045 % by mass, and a S content of not more than 0.03 % by mass.
[4] The method for producing (meth)acrylic resin according to any one of the aspects [1] to [3], wherein the polymerization reactor is made of an austenitic stainless steel with a Mo content of 0.5 to 7.0 % by mass.
[5] The method for producing (meth)acrylic resin according to any one of the aspects [1] to [3], wherein the polymerization reactor is made of an austenitic stainless steel with a Cr content of 16 to 18 % by mass, a Ni content of 10 to 14 % by mass, a Mo content of 2 to 3 % by mass, a C content of not more than 0.08 % by mass, a Si content of not more than 1 % by mass, a Mn content of not more than 2 % by mass, a P content of not more than 0.045 % by mass, and a S content of not more than 0.03 % by mass.
[6] The method for producing (meth)acrylic resin according to any one of the aspects [1] to [5], wherein the thiol chain transfer agent is 1-octanethiol.
[7] The method for producing (meth)acrylic resin according to any one of the aspects [1] to [6], wherein the radical polymerizing is carried out under conditions of a polymerization conversion ratio of 40 to 70 % by mass by a tank flow bulk polymerization method to obtain a reaction product, and the method further comprises removing an unreacted material from the reaction product.
[8] The method for producing (meth)acrylic resin according to the aspect [7], wherein the reaction product is transferred from the polymerization reactor to a vent type extruder via a pipe made of an austenitic stainless steel with a Mo content of 0.5 to 7.0 % by mass, and the removal of the unreacted material is performed in the vent type extruder.

### ADVANTAGEOUS EFFECTS OF THE INVENTION

According to the producing method of the present invention, a (meth)acrylic resin can be produced at a low cost while maintaining high transparency without being colored even in long-term production using a polymerization apparatus. Using of a methacrylic resin obtained by the producing method of the present invention can give an optical member required to have high light transmittance with a low percent defective.

### BRIEF DESCRIPTION OF THE DRAWINGS

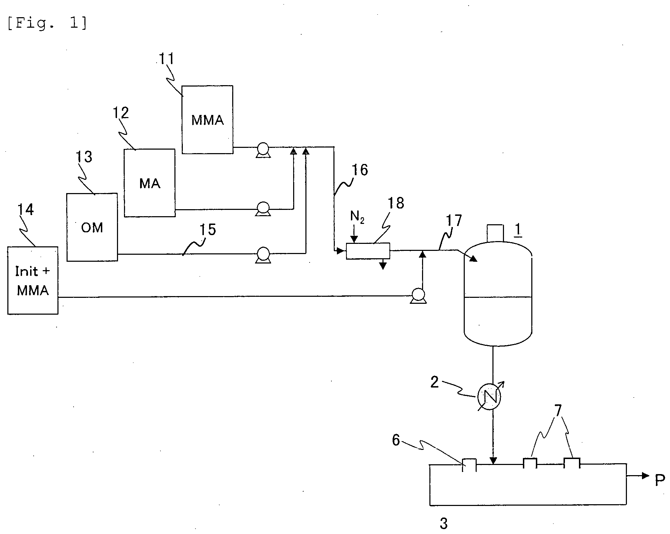

Fig. 1 shows an example of an apparatus for performing the producing method according to the present invention.

### EMBODIMENTS FOR CARRYING OUT THE INVENTION

The method for producing (meth)acrylic resin of the present invention comprises storing a thiol chain transfer agent in a tank, transferring the thiol chain transfer agent to a polymerization reactor via a pipe, and radical-polymerizing methyl methacrylate in the polymerization reactor.

For example, a polymerization apparatus shown in Fig. 1 has a tank 11, a tank 12, a tank 13, a tank 14, a N₂ mixer 18, a polymerization reactor 1, and a vent type extruder 3. Methyl methacrylate (MMA) is stored in the tank 11. A Monomer other than methyl methacrylate, such as an acrylic acid alkyl ester, is stored in the tank 12. A thiol chain transfer agent is stored in the tank 13. A radical polymerization initiator MMA solution is stored in the tank 14. In addition, the radical polymerization initiator MMA solution is prepared by dissolving a radical polymerization initiator in methyl methacrylate. The radical polymerization initiator MMA solution may contain a polymerization inhibitor in order to suppress the unexpected radical polymerization of methyl methacrylate.

The thiol chain transfer agent, the methyl methacrylate, the other monomer and the radical polymerization initiator MMA solution are transferred from each tank to the reactor 1 via the pipes, and then radical polymerization reaction is performed in the reactor 1. In the apparatus shown in Fig. 1, the thiol chain transfer agent is transferred to the reactor 1 via pipes 15, 16 and 17. The thiol chain transfer agent together with the methyl methacrylate and the other monomer passes through the pipe 16. The thiol chain transfer agent together with the radical polymerization initiator MMA solution, the methyl methacrylate and the other monomer passes through the pipe 17. Moreover, in Fig. 1, the thiol chain transfer agent, the methyl methacrylate and the other monomer are mixed, are subjected to a nitrogen gas purge in the N₂ mixer 18 to remove oxygen, and then are mixed with the radical polymerization initiator MMA solution. Alternatively, the way or the order of mixing is not particularly limited thereto, and mixing can be performed according to other manners.

In the present invention, the tank 13 for storing thiol chain transfer agent and the pipes 15, 16 and 17 through which thiol chain transfer agent passes are made of an austenitic stainless steel.

Austenitic stainless steel is a steel material comprising iron (Fe) as the major component (not less than 50 % by mass), and chromium (Cr) and nickel (Ni) as subcomponents. The austenitic stainless steel to be used in the present invention has a Cr content of preferably 16 to 18 % by mass, and a Ni content of preferably 10 to 14 % by mass. The austenitic stainless steel comprising Cr and Ni in such ranges is resistant to corrosion and has high thermal resistance.

The austenitic stainless steel to be used in the present invention has a molybdenum (Mo) content of usually 0.5 to 7.0 % by mass, and preferably 2 to 3 % by mass. The Mo content within the above range makes the steel more resistant to corrosion due to the contact with thiol chain transfer agent. This can prevent the thus obtained (meth)acrylic resin from being deteriorated in transparency and colored.

Furthermore, the austenitic stainless steel to be used in the present invention may comprise carbon (C), silicon (Si), manganese (Mn), phosphorus (P) and sulfur (S). The austenitic stainless steel to be used in the present invention has a C content of preferably not more than 0.08 % by mass, a Si content of preferably not more than 1 % by mass, a Mn content of preferably not more than 2 % by mass, a P content of preferably not more than 0.045 % by mass, and a S content of preferably not more than 0.03 % by mass.

The austenitic stainless steel to be used in the present invention preferably does not comprise copper (Cu), titanium (Ti), zirconium (Zr) and niobium (Nb) in order to avoid corrosion due to the contact with thiol chain transfer agent, but may comprise copper (Cu), titanium (Ti), zirconium (Zr) or niobium (Nb) in a small amount. When the austenitic stainless steel comprises Cu, the Cu content preferably ranges from 1.00 to 2.50 % by mass. When the austenitic stainless steel comprises Ti, the Ti content is preferably not less than the content (% by mass) corresponding to 5 times the C content. Furthermore, the austenitic stainless steel to be used in the present invention may comprise nitrogen (N) or no nitrogen (N). When N is contained, the N content ranges from preferably 0.1 to 0.22 % by mass.

The thiol chain transfer agent to be used in the present invention is a chain transfer agent comprising a compound having at least one thiol group. Examples of the thiol chain transfer agent can include an aliphatic hydrocarbon having one thiol group such as 1-octanethiol, 1-dodecanethiol, t-dodecanethiol and the like; a carboxylic acid having one thiol group such as thioacetic acid, thioglycolic acid, β-mercaptopropionic acid and the like; a carboxylic acid ester having one thiol group such as methyl-3-mercaptopropionate, 2-ethylhexyl-3-mercaptopropionate, n-octyl-3-mercaptopropionate, methoxybutyl-3-mercaptopropionate, stearyl-3-mercaptopropionate, tridecyl-3-mercaptopropionate and the like; an aliphatic hydrocarbon having two thiol groups such as 1,4-butanedithiol, 1,6-hexanedithiol, 1,10-decanedithiol and the like; and a carboxylic acid ester having not less than two thiol groups such as ethyleneglycol bisthiopropionate, butanediol bisthioglycolate, butanediol bisthiopropionate, hexanediol bisthioglycolate, hexanediol bisthiopropionate, tetraethyleneglycol-bis(3-mercaptopropionate), trimethylolpropane tristhiopropionate, tris-[(3-mercaptopropionyloxy)-ethyl]-isocyanurate, pentaerythritol tetrakis-thiopropionate, dipentaerythritol hexakis thiopropionate and the like.

Among them, an aliphatic hydrocarbon having one thiol group such as 1-octanethiol, 1-dodecanethiol and the like is preferred. These thiol chain transfer agents can be used alone or in combination of two or more. The use amount of the thiol chain transfer agent is preferably 0.08 to 1 part by mass, more preferably 0.1 to 0.8 part by mass, and further preferably 0.12 to 0.6 part by mass relative to the total 100 parts by mass of methyl methacrylate and the other monomers.

In the present invention, a monomer other than methyl methacrylate may be radicall-polymerized with methyl methacrylate. Examples of such other monomers can include, methacrylic acid esters other than methyl methacrylate, acrylic acid esters, methacrylic acid, acrylic acid, acrylonitrile, methacrylonitrile, styrene, acrylamide, methacrylamide and the like. Among them, an acrylic acid alkyl ester is preferably used in the present invention. In addition, alkyl is a group in which one hydrogen atom is eliminated from an acyclic saturated hydrocarbon or a cyclic saturated hydrocarbon. Alkyl may also have a substituent such as an epoxy group, a vinyl group, an alkoxy group, an aryloxy group, a hydroxy group, an aryl group and the like.

Examples of the acrylic acid alkyl esters to be used in the present invention can include methyl acrylate, ethyl acrylate, n-propyl acrylate, isopropyl acrylate, n-butyl acrylate, isobutyl acrylate, s-butyl acrylate, t-butyl acrylate, amyl acrylate, isoamyl acrylate, n-hexyl acrylate, 2-ethylhexyl acrylate, pentadecyl acrylate, dodecyl acrylate, cyclohexyl acrylate, norbornenyl acrylate, isobornyl acrylate, benzyl acrylate, phenoxyethyl acrylate, 2-hydroxyethyl acrylate, 2-ethoxyethyl acrylate, glycidyl acrylate, allyl acrylate and the like. In these examples, methyl acrylate is preferred.

The amount of monomers other than methyl methacrylate is preferably 0 to 50 parts by mass, more preferably 0.01 to 40 parts by mass, further preferably 0.05 to 30 parts by mass, and even more preferably 0.1 to 20 parts by mass relative to the total 100 parts by mass of methyl methacrylate and the other monomers.

Radical polymerization can be performed in the presence of a radical polymerization initiator. Examples of the radical polymerization initiator are not particularly limited, as long as it generates a reactive radical. Examples of the radical polymerization initiator can include t-hexylperoxyisopropyl monocarbonate, t-hexylperoxy 2-ethylhexanoate, 1,1,3,3-tetramethylbutylperoxy 2-ethylhexanoate, t-butylperoxypivalate, t-hexylperoxypivalate, t-butylperoxyneodecanoate, t-hexylperoxyneodecanoate, 1,1,3,3-tetramethylbutylperoxyneodecanoate, 1,1-bis(t-hexylperoxy)cyclohexane, benzoyl peroxide, 3,5,5-trimethylhexanoyl peroxide, lauroyl peroxide, 2,2'-azobis(2-methylpropionitrile), 2,2'-azobis(2-methylbutyronitrile), dimethyl 2, 2'-azobis(2-methylpropionate) and the like. In these examples, t-hexylperoxy 2-ethylhexanoate, 1,1-bis(t-hexylperoxy)cyclohexane, and 2,2'-azobis(2-methylpropionitrile) are preferred.

Radical polymerization can be performed by a suspension polymerization method, a solution polymerization method, a bulk polymerization method, an emulsion polymerization method, or the like. In the present invention, radical polymerization is preferably performed by a bulk polymerization method in order to inhibit the transparency from decreasing due to coloring or the like.

As polymerization operation, mentioned are, so-called batch operation by which charging of raw materials, polymerization reaction, and discharging of product are performed in sequence, and so-called flow operation by which feeding of raw materials, polymerization reaction, and withdrawing of product are performed simultaneously. In the present invention, the flow operation is preferably employed.

In accordance with the flow operation, a tank reactor with which a reaction can be performed under near complete mixing conditions, a tube reactor with which a reaction can be performed under near plug flow conditions or others can be used, for example. In the present invention, the tank reactor is preferably used.

When radical polymerization is performed by the bulk polymerization method, the polymerization conversion ratio is set at preferably 40 to 70 % by mass, and more preferably 42 to 65 % by mass.

In the present invention, radical polymerization is most preferably performed by a tank-flow bulk polymerization method under conditions of the polymerization conversion ratio of 40 to 70 % by mass.

A polymerization reactor has a container being a reaction field, stirring facilities, a feed port for feeding raw materials to the container, a withdrawing port for withdrawing product. Examples of stirring facilities in a tube reactor can include a static mixer, a dynamic mixer and the like. Examples of a stirring facility of a tank reactor can include a Max blend stirrer, a lattice-shaped impellers stirrer, a propeller stirrer, a screw stirrer, a helical ribbon stirrer, a paddle stirrer and the like. In these examples, the Max blend stirrer is preferred in terms of homogeneous mixing performance.

The polymerization reactor to be used in the present invention is preferably made of the above austenitic stainless steel. In particular, portions, with which raw materials comprising a thiol chain transfer agent or a product considered to contain the thiol chain transfer agent remaining therein comes into contact, are preferably made of the above austenitic stainless steel.

Volatile matters such as unreacted materials contained in the reaction product can be recovered by a known means in chemical engineering. As a preferable recovering method, mentioned can be, for instance, a method for removing volatile matters by heating. As the method for removing volatile matters by heating, mentioned can be an equilibrium flash evaporation method and an adiabatic flash evaporation method. And the adiabatic flash evaporation method is preferred.

In the present invention, first, a withdrawn reaction product is heated in a heat exchanger 2. As a heat source of the heat exchanger 2, steam generated by a boiler or another apparatus can be used. Moreover, vapor of the volatile matters evaporated from the reaction product as described later can also be used as the heat source. Moreover, pressure of a reaction product is increased using a pump or the like in order to enhance the efficiency of flash evaporation. In the present invention, pipes that run from the polymerization reactor to the heat exchanger and the heat exchanger are preferably made of the above austenitic stainless steel.

Next, the heated reaction product may be introduced into a depressurized tank to be flash-evaporated. The temperature at which the adiabatic flash evaporation method is performed is preferably 200 to 300°C, and more preferably 220 to 270°C. When the temperature for performing the adiabatic flash evaporation method is lower than 200°C, removal of volatile matters takes long time, and volatile matters are removed insufficiently, which can result in poor appearance such as silver streaks in the shaped product. On the other hand, when the temperature for performing the adiabatic flash evaporation method exceeds 300°C, the (meth)acrylic resin tends to be colored due to oxidation, burning, and the like. The adiabatic flash evaporation method may be performed in multiple stages. The reaction product flowing through a heat-transfer tube is heated by the vapor of unreacted materials evaporated by the flash evaporation, and then the thus heated reaction product is fed into a low-pressure flash tank to be flash-evaporated. The flash tank, with which a product considered to contain the thiol chain transfer agent remaining therein comes into contact, is preferably made of the above austenitic stainless steel.

Volatile matters can be removed by a vent type extruder 3. The vent type extruder 3 generally has a reaction product feed port capable of feeding a reaction product comprising polymers and volatile matters, a polymer discharge port capable of discharging polymers separated from the reaction product, at least one vent capable of discharging volatile matters separated from the reaction product, and a screw for transferring the reaction product from the reaction product feed port to the polymer discharge port while kneading it. A vent 7 located on the position closer to the polymer discharge port than to the reaction product feed port is referred to as "front vent", and a vent 6 located on the position more distant from the polymer discharge port than from the reaction product feed port is referred to as "rear vent". The vent type extruder to be used in the present invention is equipped with an additive input port located on the position closer to the polymer discharge port than to the front vent located closest to the polymer discharge port. In the extruder preferably under reduced pressure, the fed reaction product is preferably subjected to flash evaporation at the reaction product feed port. Volatile matters are then evaporated while being transferred by the screw. The evaporated volatile matters are discharged from the vent. The extruder may be a single screw extruder or a twin screw extruder, for example. A screw is generally divided into a feed zone, a compression zone, a metering zone and a mixing zone, but zones are not particularly limited thereto. In addition, in the mixing zone, variously shaped screws such as those of dulmage type, rotor type, flute mixing type, and the like, including convexo-concave, groove-shaped, and pin-shaped screws, can be used in combination as appropriate. In the present invention, the pipes that run from the polymerization reactor to the vent type extruder are preferably made of the above austenitic stainless steel.

Volatile matters immediately after the recovery thereof by the above method for removing volatile matters may contain, in addition to methyl methacrylate, an acrylic acid alkyl ester or a chain transfer agent, dimers or trimers. Such dimers or trimers may affect the properties of (meth)acrylic resin and thus are preferably removed from the recovered volatile matters. Upon removal of dimers or trimers, a part of a chain transfer agent or a solvent may be removed.

Dimers or trimers can be removed by a known means of chemical engineering. For example, a method that involves distillation is preferable. A distillation column to be used in the present invention is not particularly limited, but the column is preferably a multistage distillation column having a number of stages of about 6 to 20, and a reflux ratio of about 0.4 to 2.0. The amount of unreacted materials remaining in the (meth)acrylic resin obtained by the present invention is preferably not more than 1 % by mass, and more preferably not more than 0.5 % by mass.

An additive can be added to a reaction product from which volatile matters have been removed. Examples of the additive can include lubricants (e.g., higher alcohols, glycerine monoesters and the like), a processing aid, a light stabilizer, an antioxidant, a plasticizer, a UV absorber, an impact resistance agent, a foaming agent, a filler, a coloring agent, an anti-static agent, a light diffusing agent and the like.

A polymer discharge port of an extruder may be equipped with a breaker plate, a screen, or the like for removing foreign substances, carbonized substances, gel substances, and the like. The breaker plate is generally a disk wherein a plurality of holes with diameters of 3 to 4 mm are bored concentrically. The screen is produced by overlaying 1 to several sheets of wire mesh with aperture sizes varied depending on applications or purposes.

The (meth)acrylic resin obtained by the producing method of the present invention can be processed into pellets, powders, granules, or the like in order to facilitate the handling as a molding material according to a known method.

The (meth)acrylic resin obtained by the producing method of the present invention can be molded (melt-heat molding) by a conventionally known molding method such as injection molding, compression molding, extrusion, and vacuum molding, so that various shaped products can be obtained. Examples of the shaped products made of the (meth)acrylic resin can include: signboard parts such as advertising pillars, stand signboards, side signboards, transom signboards, roof signboards and the like; display parts such as showcases, partition panels, store displays and the like; illumination parts such as fluorescent lamp covers, mood lighting covers, lamp shades, luminous ceilings, luminous walls, chandeliers and the like; interior parts such as pendants, mirrors and the like; architectural parts such as doors, domes, safety window glass, partitions, stair wainscots, balcony wainscots, leisure building roofs and the like; transportation related parts such as air plane windshields, pilot visors, motorcycle and motorboat windshields, bus sun visors, automobile side visors, rear visors, head wings, headlight covers and the like; electronic equipment parts such as faceplates for audiovisuals, stereo covers, television protective masks, vending machines and the like; medical equipment parts such as incubators, roentgen parts and the like; instrument related parts such as machine covers, instrument covers, experiment devices, rulers, dials, observation windows and the like; optics related parts such as liquid crystal protective plates, light guide plates, light guide films, Fresnel's lens, lenticular lens, front panels of various displays, diffusion boards and the like; traffic related parts such as road signs, direction boards, traffic mirrors, noise barriers and the like; film members such as polarizer protective films, polarizing plate protective films, phase difference films, surface materials for automobile interior, surface materials for cell phones, marking films and the like; materials for household electrical appliances, such as washing machine canopies, control panels, top surface panels for rice cookers and the like; and others, such as greenhouses, large-sized water tanks, box-type water tanks, clock panels, bath tubs, sanitary, desk mats, parts of recreational devices, toys, face protecting masks when welding and the like.

### EXAMPLES

Below, the present invention is more specifically described with reference to Examples and Comparative Examples. However, the present invention shall not be limited to the following examples.

### (Polymerization conversion ratio)

A column (GLC-G-230 Sciences Inc., INERT CAP 1 (df = 0.4 µm, 0.25 mm I.D. 60 m in length)) was connected to a gas chromatography system (Shimadzu Corporation, GC-14A), and reaction products discharged continuously from a polymerization reactor were analyzed under the following conditions: the injection temperature was 180°C; the detector temperature was 180°C; the column temperature was increased from 60°C to 200°C at a rate of temperature increase of 10°C/min.

### (Preparation of plate)

A plate with a length of 200 mm, a width of 60 mm and a thickness of 6 mm was obtained by performing injection molding using an injection molding machine (The Japan Steel Works, Ltd., J-110ELIII) under conditions of a cylinder temperature of 280°C, a mold temperature of 60°C, and a molding cycle of 4 minutes.

### (Light transmittance)

Light transmittance at a wavelength of 450 nm in the longitudinal direction (200 mm) of the above-obtained plate (200 mm in length, 60 mm in width, and 6 mm in thickness) was determined with a PC-2200 spectrophotometer (Shimadzu Corporation).

### (Yellow index (YI))

The above-obtained plate (200 mm in length, 60 mm in width, and 6 mm in thickness) was measured for yellow index under the following conditions. Light transmittance in a light path length of 200 mm was measured using a spectrophotometer (Shimadzu Corporation, PC-2200) and the C illuminant every 1 nm within the wavelength range of 340 nm to 700 nm. An XYZ value was found in accordance with the method described in JIS Z-8722 from the thus obtained measurement values and then Yellow index (YI) was calculated in accordance with the method described in JIS K-7105.

### Example 1

Apparatuses to be used for the flow shown in Fig. 1 were prepared. A tank 11, a tank 12, a tank 13, and a tank 14, and all pipes running from each tank to a reactor 1 were produced with an austenitic stainless steel having the composition ratios shown in Table 1.

### [Tab. 1]

**Table 1**

| | C | Si | Mn | P | S | Ni | Cr | Mo | Cu | N | others |
|---|---|---|---|---|---|---|---|---|---|---|---|
| Ex.1 | 0.08≧ | 1.00≧ | 2.00≧ | 0.045≧ | 0.030≧ | 10.00-14.00 | 16.00-18.00 | 2.00-3.00 | - | - - | - |
| Comp.Ex.1 | 0.08≧ | 1.00≧ | 2.00≧ | 0.045≧ | 0.030≧ | 8.00-10.50 | 18.00-20.00 | - | - | - | - |

| | | | | | | | | | | | |
|---|---|---|---|---|---|---|---|---|---|---|---|
| Unit: % by mass | | | | | | | | | | | |

Methyl methacrylate (MMA) was stored in the tank 11, methyl acrylate (MA) was stored in the tank 12, 1-octanethiol (thiol chain transfer agent) was stored in the tank 13, and 2,2'-azobis2-methylpropionitrile (radical polymerization initiator) MMA solution was stored in the tank 14.

From the tank 11, the tank 12, and the tank 13, methyl methacrylate (73.6 kg), methyl acrylate (6.4 kg), and 1-octanethiol (360 g) were transferred to and charged into a flow tank reactor 1 (capacity: 0.2 m³, tank diameter: 500 mm, Max blend blade, blade diameter: 260 mm, number of revolutions: 200 rpm) equipped with a brine cooling condenser. The air within the reactor was replaced by nitrogen. Subsequently, the temperature was increased to 140°C.

Methyl methacrylate, methyl acrylate, 1-octanethiol, and 2,2'-azobis2-methylpropionitrile MMA solution were fed from the tank 11, the tank 12, the tank 13, and the tank 14 to the reactor while maintaining 140°C with proportions of 92 parts by mass of methyl methacrylate, 8 parts by mass of methyl acrylate, 0.45 part by mass of 1-octanethiol, and 0.0065 part by mass of 2,2'-azobis2-methylpropionitrile at a flow rate achieving the mean residence time of 2.5 hours in the reactor. Simultaneously with feeding of the raw materials, nitrogen was fed to an N₂ mixer 18, so as to remove oxygen contained in methyl methacrylate, methyl acrylate and 1-octanethiol. Furthermore, simultaneously with the feeding of the raw materials, liquid was withdrawn from the bottom of the reactor 1, keeping the liquid level height within the reactor at a level.

The liquid withdrawn from the reactor 1 was fed to a heater 2, and then the temperature was increased to 230°C. Subsequently, the liquid was fed at a constant flow rate to a twin screw extruder 3 regulated at 250°C. In the twin screw extruder, volatile matters mainly comprising unreacted monomers were separated and removed, while resin component P was extruded in a strand form. The strand was cut with a pelletizer to continuously obtain pelleted (meth)acrylic resin.

One day after the beginning of the continuous operation, a reaction product solution was sampled using the reactor sampling tube. The polymerization conversion ratio was calculated by the above method. The polymerization conversion ratio; that is, the content of the (meth)acrylic resin in the reaction product solution was 52 % by mass.

A plate was obtained by the above method using the pelleted (meth)acrylic resin obtained one day after the beginning of the continuous operation, and then measured for light transmittance and yellow index. The results are shown in Table 2.

In accordance with the amounts of methyl methacrylate, methyl acrylate, 1-octanethiol and 2,2'-azobis2-methylpropionitrile MMA solution consumed, each tank was supplemented with new methyl methacrylate, methyl acrylate, 1-octanethiol and 2,2'-azobis2-methylpropionitrile MMA solution.

One year after the beginning of the above continuous operation, the above feeding of raw materials was stopped, and the liquid was completely withdrawn from the reactor. With the use of the thus withdrawn pelleted (meth)acrylic resin, a plate was obtained by the above method, and then measured for light transmittance and yellow index. The results are shown in Table 2.

After continuous operation was completely stopped, the inner wall surfaces of the tank 11 in which 1-octanethiol had been stored, the inner surfaces of pipes through which 1-octanethiol had passed, and the inner wall surfaces of the reactor were visually observed. No corrosion such as rust was observed on all inner surfaces.

### Example 2

A (meth)acrylic resin was obtained in the same manner as in Example 1 except that the amount of methyl acrylate was changed to 0 part by mass. Furthermore, in accordance with the same method as that in Example 1, plates were obtained from a pelleted (meth)acrylic resin obtained one day after the beginning of continuous operation and a pelleted (meth)acrylic resin obtained one year after the beginning of continuous operation, and then measured for light transmittance and yellow index. The results are shown in Table 2.

After continuous operation was completely stopped, the inner wall surfaces of the tank 11 in which 1-octanethiol had been stored, the inner surfaces of pipes through which 1-octanethiol had passed, and the inner wall surfaces of the reactor were visually observed. No corrosion such as rust was observed on all inner surfaces.

### Comparative example 1

A (meth)acrylic resin was obtained in the same manner as in Example 1 except that the tank 11, the tank 12, the tank 13, and the tank 14 and all pipes running from each tank to the reactor 1 were produced with the austenitic stainless steel with the composition ratios as in Table 1. Furthermore, plates were obtained by the same method as that in Example 1, from a pelleted (meth)acrylic resin obtained one day after the beginning of continuous operation and a pelleted (meth)acrylic resin obtained one year after the beginning of continuous operation, and then measured for light transmittance and yellow index. The results are shown in Table 2.

After continuous operation was completely stopped, the inner wall surfaces of the tank 11 in which 1-octanethiol had been stored, the inner surfaces of pipes through which 1-octanethiol had passed, and the inner wall surfaces of the reactor were visually observed. Welds slightly rusted.

### [Table 2]

**Table 2**

| | Ex. | Comp.Ex. | Ex. |
|---|---|---|---|
| | 1 | 1 | 2 |
| One day after beginning | | | |
| light transmittance[%] | 86.2 | 86.1 | 86.2 |
| YI | 4.6 | 4.7 | 4.6 |

| One year after beginning | | | |
|---|---|---|---|
| corrosion | No | Yes | No |
| light transmittance[%] | 86.2 | 83.6 | 86.2 |
| YI | 4.6 | 8.9 | 4.6 |

As described above, a tank for storing a thiol chain transfer agent, and pipes for transferring the thiol chain transfer agent are produced with the austenitic stainless steel with a Mo content of 0.5 to 7.0 % by mass, so that a (meth)acrylic resin can be produced at a low cost while maintaining high transparency even in long-term production.

## Claims

1. A method for producing (meth)acrylic resin, the method comprising storing a thiol chain transfer agent in a tank made of an austenitic stainless steel with a Mo content of 0.5 to 7.0 % by mass, transferring the thiol chain transfer agent to a polymerization reactor via a pipe made of an austenitic stainless steel with a Mo content of 0.5 to 7.0 % by mass, and radical-polymerizing methyl methacrylate in the polymerization reactor.

2. The method for producing (meth) acrylic resin according to claim 1, wherein, in the radical-polymerizing, an acrylic acid alkyl ester is radical-polymerized together with the methyl methacrylate.

3. The method for producing (meth)acrylic resin according to claim 1 or 2, wherein the tank and the pipe are made of an austenitic stainless steel with a Cr content of 16 to 18 % by mass, a Ni content of 10 to 14 % by mass, a Mo content of 2 to 3 % by mass, a C content of not more than 0.08 % by mass, a Si content of not more than 1 % by mass, a Mn content of not more than 2 % by mass, a P content of not more than 0.045 % by mass, and a S content of not more than 0.03 % by mass.

4. The method for producing (meth)acrylic resin according to any one of claims 1 to 3, wherein the polymerization reactor is made of an austenitic stainless steel with a Mo content of 0.5 to 7.0 % by mass.

5. The method for producing (meth)acrylic resin according to any one of claims 1 to 3, wherein the polymerization reactor is made of an austenitic stainless steel with a Cr content of 16 to 18 % by mass, a Ni content of 10 to 14 % by mass, a Mo content of 2 to 3 % by mass, a C content of not more than 0.08 % by mass, a Si content of not more than 1 % by mass, a Mn content of not more than 2 % by mass, a P content of not more than 0.045 % by mass, and a S content of not more than 0.03 % by mass.

6. The method for producing (meth)acrylic resin according to any one of claims 1 to 5, wherein the thiol chain transfer agent is 1-octanethiol.

7. The method for producing (meth)acrylic resin according to any one of claims 1 to 6, wherein the radical-polymerizing is carried out under condition of a polymerization conversion ratio of 40 to 70 % by mass by a tank-flow bulk-polymerization method to obtain a reaction product, and the method further comprises removing an unreacted material from the reaction product.

8. The method for producing (meth)acrylic resin according to claim 7, wherein the reaction product is transferred from the polymerization reactor to a vent type extruder via a pipe made of an austenitic stainless steel with a Mo content of 0.5 to 7.0 % by mass, and the removal of the unreacted material is performed in the vent type extruder.

## Patentansprüche

1. Verfahren zur Herstellung von (Meth)acrylharz, wobei das Verfahren das Lagern eines Thiolkettenübertragungsreagenzes in einem Tank, hergestellt aus einem austenitischen Edelstahl mit einem Mo-Gehalt von 0,5 bis 7,0 Massen-%, das Transferieren des Thiolkettenübertragungsreagenzes zu einem Polymerisationsreaktor über ein Rohr, hergestellt aus einem austenitischen Edelstahl mit einem Mo-Gehalt von 0,5 bis 7,0 Massen-%, und das radikalische Polymerisieren von Methylmethacrylat im Polymerisationsreaktor umfasst.

2. Verfahren zur Herstellung von (Meth)acrylharz nach Anspruch 1, wobei beim radikalischen Polymerisieren ein Acrylsäurealkylester zusammen mit dem Methylmethacrylat radikalisch polymerisiert wird.

3. Verfahren zur Herstellung von (Meth)acrylharz nach Anspruch 1 oder 2, wobei der Tank und das Rohr aus einem austenitischen Edelstahl mit einem Cr-Gehalt von 16 bis 18 Massen-%, einem Ni-Gehalt von 10 bis 14 Massen-%, einem Mo-Gehalt von 2 bis 3 Massen-%, einem C-Gehalt von nicht mehr als 0,08 Massen-%, einem Si-Gehalt von nicht mehr als 1 Massen-%, einem Mn-Gehalt von nicht mehr als 2 Massen-%, einem P-Gehalt von nicht mehr als 0,045 Massen-% und einem S-Gehalt von nicht mehr als 0,03 Massen-% hergestellt sind.

4. Verfahren zur Herstellung von (Meth)acrylharz nach einem der Ansprüche 1 bis 3, wobei der Polymerisationsreaktor aus einem austenitischen Edelstahl mit einem Mo-Gehalt von 0,5 bis 7,0 Massen-% hergestellt ist.

5. Verfahren zur Herstellung von (Meth)acrylharz nach einem der Ansprüche 1 bis 3, wobei der Polymerisationsreaktor aus einem austenitischen Edelstahl mit einem Cr-Gehalt von 16 bis 18 Massen-%, einem Ni-Gehalt von 10 bis 14 Massen-%, einem Mo-Gehalt von 2 bis 3 Massen-%, einem C-Gehalt von nicht mehr als 0,08 Massen-%, einem Si-Gehalt von nicht mehr als 1 Massen-%, einem Mn-Gehalt von nicht mehr als 2 Massen-%, einem P-Gehalt von nicht mehr als 0,045 Massen-% und einem S-Gehalt von nicht mehr als 0,03 Massen-% hergestellt ist.

6. Verfahren zur Herstellung von (Meth)acrylharz nach einem der Ansprüche 1 bis 5, wobei das Thiolkettenübertragungsreagenz 1-Octanthiol ist.

7. Verfahren zur Herstellung von (Meth)acrylharz nach einem der Ansprüche 1 bis 6, wobei das radikalische Polymerisieren unter der Bedingung eines Polymerisationsumsetzungsrate von 40 bis 70 Massen-% durch ein Tankflußmassepolymerisationsverfahren durchgeführt wird, um ein Reaktionsprodukt zu erhalten, und das Verfahren weiter das Entfernen eines nicht umgesetzten Materials aus dem Reaktionsprodukt umfasst.

8. Verfahren zur Herstellung von (Meth)acrylharz nach Anspruch 7, wobei das Reaktionsprodukt aus dem Polymerisationsreaktor über ein Rohr, hergestellt aus einem austenitischen Edelstahl mit einem Mo-Gehalt von 0,5 bis 7,0 Massen-%, in einen Entgasungsextruder transferiert wird und die Entfernung des nicht umgesetzten Materials im Entgasungsextruder durchgeführt wird.

## Revendications

1. Procédé de production de résine (méth)acrylique, le procédé comprenant le stockage d'un agent de transfert de chaîne thiol dans un réservoir en acier inoxydable austénitique avec une teneur en Mo allant de 0,5 à 7,0% en masse, le transfert de l'agent de transfert de chaîne thiol à un réacteur de polymérisation par un tuyau en acier inoxydable austénitique avec une teneur en Mo allant de 0,5 à 7,0% en masse et la polymérisation par voie radicalaire du méthyl méthacrylate dans le réacteur de polymérisation.

2. Procédé de production de résine (méth)acrylique selon la revendication 1, dans lequel, lors de la polymérisation par voie radicalaire, un alkylester d'acide acrylique est polymérisé par voie radicalaire avec le méthyl méthacrylate.

3. Procédé de production de résine (méth)acrylique selon la revendication 1 ou 2, dans lequel le réservoir et le tuyau sont en acier inoxydable austénitique avec une teneur en Cr allant de 16 à 18% en masse, une teneur en Ni allant de 10 à 14% en masse, une teneur en Mo allant de 2 à 3% en masse, une teneur en C de pas plus de 0,08% en masse, une teneur en Si de pas plus de 1% en masse, une teneur en Mn de pas plus de 2% en masse, une teneur en P de pas plus de 0,045% en masse et une teneur en S de pas plus de 0,03% en masse.

4. Procédé de production de résine (méth)acrylique selon l'une quelconque des revendications 1 à 3, dans lequel le réacteur de polymérisation est en acier inoxydable austénitique avec une teneur en Mo allant de 0,5 à 7,0% en masse.

5. Procédé de production de résine (méth)acrylique selon l'une quelconque des revendications 1 à 3, dans lequel le réacteur de polymérisation est en acier inoxydable austénitique avec une teneur en Cr allant de 16 à 18% en masse, une teneur en Ni allant de 10 à 14% en masse, une teneur en Mo allant de 2 à 3% en masse, une teneur en C de pas plus de 0,08% en masse, une teneur en Si de pas plus de 1% en masse, une teneur en Mn de pas plus de 2% en masse, une teneur en P de pas plus de 0,045% en masse et une teneur en S de pas plus de 0,03% en masse.

6. Procédé de production de résine (méth)acrylique selon l'une quelconque des revendications 1 à 5, dans lequel l'agent de transfert de chaîne thiol est le 1-octanethiol.

7. Procédé de production de résine (méth)acrylique selon l'une quelconque des revendications 1 à 6, dans lequel la polymérisation par voie radicalaire s'effectue à la condition d'un rapport de conversion de polymérisation de 40 à 70% en masse par un procédé de polymérisation de masse à débit de réservoir pour obtenir un produit réactionnel, et le procédé comprend en outre l'élimination d'une matière n'ayant pas réagi du produit réactionnel.

8. Procédé de production de résine (méth)acrylique selon la revendication 7, dans lequel le produit réactionnel est transféré du réacteur de polymérisation à une extrudeuse à ouverture via un tuyau en acier inoxydable austénitique avec une teneur en Mo allant de 0,5 à 7,0% en masse, et l'élimination de la matière n'ayant pas réagi est effectuée dans l'extrudeuse à ouverture.
